# EUROPEAN PATENT APPLICATION

(11) **EP 4 474 019 A1**
(43) Date of publication of application: **11.12.2024**
(21) Application number: 23749561.9
(22) Date of filing: 20.01.2023
(51) Int. Cl.: A61Q 1/00, A61Q 17/04, A61K 8/06, A61K 8/19, A61K 8/26, A61K 8/27

(54) **WATER-IN-OIL EMULSION COSMETIC**

(30) Priority: 02.02.2022 JP 2022015229
(71) Applicant: Shiseido Company, Ltd., Chuo-ku Tokyo 104-0061 (JP)
(72) Inventor: NISHI, Haruka, Tokyo 104-0061 (JP); UJIMOTO, Kei, Tokyo 104-0061 (JP)
(74) Representative: Ter Meer Steinmeister & Partner
(86) International application number: PCT/JP2023/001788
(87) International publication number: WO 2023/149245

(57) **Abstract**

[Problem] An objective of the present invention is to provide a water-in-oil emulsion cosmetic that does not tend to form color streaks (streaks caused by pigments becoming non-uniform), that has excellent formulation stability and that has smooth properties in use. [Solution] The water-in-oil emulsion cosmetic of the present invention is characterized by comprising (A) an amino acid-treated pigment, (B) an organically modified clay mineral, and (C) an oil gelling agent other than component (B).

## Description

### TECHNICAL FIELD

The present invention relates to a water-in-oil emulsion cosmetic that does not tend to form color streaks (streaks caused by pigments becoming non-uniform), that has excellent formulation stability and that has smooth properties in use.

### BACKGROUND ART

In pigment powders that are blended into cosmetics, particularly makeup cosmetics such as foundations and eye makeup, sunscreen cosmetics, etc., surface hydrophobization treatments are indispensable for improving the water resistance and properties in use of the cosmetics.

For example, Patent Document 1 describes that, when a pigment surface-treated with an N-acylamino acid or a salt thereof was used in a water-in-oil emulsion cosmetic, there were problems in that line-shaped non-uniformities were observed at the beginning of application and that patchiness occurred over time. In order to solve these problems, Patent Document 1 describes that a water-in-oil emulsion cosmetic in which these problems are solved was obtained by making combined use of an ester oil selected from among pentaerythritol fatty acid esters and dipentaerythritol fatty acid esters that are liquid at 25 °C.

As mentioned above, there are cases in which new problems occur when specific surface-hydrophobized pigments are used in cosmetics.

### RELATED ART

### PATENT DOCUMENTS

Patent Document 1: JP 2019-147773 A

### SUMMARY OF THE INVENTION

### PROBLEM TO BE SOLVED BY THE INVENTION

In the process of research by the present inventors, it became clear that water-in-oil emulsion cosmetics have the problem that, when amino acid-treated pigments are used, the dispersibility of the pigments becomes low, the pigments aggregate with each other and color streaks (streaks formed by pigments becoming non-uniform) tend to form in comparison with the case in which silicone-treated pigments are used. Additionally, when the viscosity of the system itself was increased in order to suppress the formation of color streaks, there was a problem in that the properties in use of the cosmetic were lowered, such as the spreadability becoming worse.

Thus, an objective of the present invention is to provide a water-in-oil emulsion cosmetic in which an amino acid-treated pigment is blended, the water-in-oil emulsion cosmetic not forming color streaks, having excellent formulation stability, and having smooth properties in use.

### MEANS FOR SOLVING THE PROBLEM

The inventors carried out diligent investigations towards solving the aforementioned problem, as a result of which they discovered that, in a water-in-oil emulsion cosmetic in which an amino acid-treated pigment is blended, blending a combination of an organically modified clay mineral with a specific oil gelling agent results in a water-in-oil emulsion cosmetic in which the formation of color streaks is suppressed, the formulation stability over time is improved and smooth properties in use are obtained, thereby completing the present invention.

The present invention provides a water-in-oil emulsion cosmetic comprising:
(A) an amino acid-treated pigment;
(B) an organically modified clay mineral; and
(C) an oil gelling agent other than component (B).

### EFFECTS OF THE INVENTION

The cosmetic according to the present invention, by having the above-mentioned configuration, can provide a water-in-oil emulsion cosmetic in which an amino acid-treated pigment is blended, wherein the formation of color streaks is sufficiently suppressed while also having excellent formulation stability over time, and having smooth properties in use.

The "color streaks" in the present specification refer to streaks that are formed by the occurrence of powder aggregation caused by the pigment having low dispersibility and the pigment becoming non-uniform.

### MODES FOR CARRYING OUT THE INVENTION

The water-in-oil emulsion cosmetic according to the present invention is characterized by comprising (A) an amino acid-treated pigment, (B) an organically modified clay mineral, and (C) an oil gelling agent other than component (B). Hereinafter, the respective components constituting the cosmetic of the present invention will be explained in detail.

### <(A) Amino acid-treated pigment>

The (A) amino acid-treated pigment (hereinafter sometimes referred to simply as "component (A)") that is blended into the cosmetic according to the present invention refers to an inorganic pigment that is surface-treated with a surface treatment agent containing an amino acid or a salt thereof. By using a specific compound as the surface treatment agent, a cosmetic with excellent powder dispersibility is obtained. As the surface treatment agent for the pigment in the present invention, the amino acids mentioned below or salts thereof may be used, or another compound that is generally used as a surface treatment agent for powders may be further included in addition to the amino acids mentioned below or salts thereof.

Specific examples of the pigments include red iron oxide (Bengal red), iron titanate, γ-iron oxide, yellow iron oxide, ocher, black iron oxide, carbon black, lower titanium oxides, mango violet, cobalt violet, chromium oxide, chromium hydroxide, cobalt titanate, ultramarine blue, Prussian blue, etc. In particular, a pigment-grade iron oxide such as yellow iron oxide, red iron oxide or black iron oxide, a pigment-grade titanium oxide, etc. is preferably used as the pigment in the present invention. In this case, pigment grade means that the average particle size is 100 nm to 1 µm.

Examples of the "amino acid" used as the surface treatment agent for component (A) include glutamic acid, aspartic acid, lysine etc., among which glutamic acid and aspartic acid are preferred. Additionally, the "amino acid" may be an "amino acid acylated by a saturated fatty acid" in which an acyl group, preferably a saturated fatty acid having 12 to 20 carbon atoms, is condensed on an amino group in the amino acid. Examples of the "acyl group" include stearoyl groups, lauroyl groups, etc. The "salt" may be selected from among alkali metal salts such as sodium and potassium, and alkaline earth metal salts, among which sodium salts are preferred. Specific examples of acylated amino acids include disodium stearoyl glutamate, sodium lauroyl glutamate, sodium lauroyl aspartate and lauroyl lysine.

The powder dispersibility can be further improved by adding an "ester" to the aforementioned "amino acid or a salt thereof" as the surface treatment agent for component (A) in the present invention. The "ester" is a compound obtained by a monovalent or divalent fatty acid having 8 to 12 carbon atoms and a saturated fatty alcohol having 12 to 20 carbon atoms being linked by an ester bond, wherein the alkyl chains in the fatty acid and in the fatty alcohol may be linear or branched. In particular, isostearyl sebacate is preferably used.

The (A) amino acid-treated pigment in the present invention can be prepared by a conventional method, by adsorbing an amino acid or a salt thereof to the pigment surfaces.

The surface treatment agent in the (A) amino acid-treated pigment used in the present invention is preferably a surface treatment agent containing an amino acid selected from between disodium stearoyl glutamate and sodium lauroyl glutamate, and a surface treatment agent (NHS treatment agent) containing disodium stearoyl glutamate and isostearyl sebacate is more preferable.

A commercially available product may be used as the (A) amino acid-treated pigment in the present invention, and preferable commercially available products include, for example, ASL-treated powders such as ASL-1 TiO2 CR-50, ASL-Red R516P, ASL-Yellow LL-100P and ASL-Black BL-100P; ASI-treated powders such as ASI TiO2 CR-50, ASI-Red R516P, ASI-Yellow LL-100P, ASI- Black BL-100P and ASI-Talc JA46R (all of the above manufactured by Daito Kasei Kogyo Co., Ltd.); and NHS-treated powders such as NHS-Titanium CR-50, NHS-Red R516PS, NHS-Yellow LL-100P, NHS-Black BL-100P, NHS-Mica M-102 and NHS-Talc JA-46R (all of the above manufactured by Miyoshi Kasei, Inc.).

As component (A) in the present invention, one or more pigments treated with the surface treatment agents mentioned above may be combined and blended.

The total blended amount of component (A) is not particularly limited as long as the desired coloration is obtained, but should normally be at least 0.1% by mass, for example, 1% to 20% by mass, preferably 2% to 10% by mass relative to the overall amount of the cosmetic. If the blended amount is less than 0.1% by mass, sufficient coloration cannot be obtained, and if 20% by mass is exceeded, the stability tends to be poor.

In the cosmetic of the present invention, sufficient stability is obtained by components (B) and (C) described below. Therefore, it is possible to blend 4% or more of component (A) relative to the overall amount of the cosmetic.

### <(B) Organically modified clay mineral>

As the (B) organically modified clay mineral (hereinafter sometimes referred to simply as "component (B)") blended into the cosmetic according to the present invention, it is possible to use a clay mineral represented by the following general formula (1), which is a type of colloidal hydrated ammonium silicate having a three-layered structure, modified by a quaternary ammonium salt type cationic surfactant.

(X,Y)₂₋₃(Si,Al)₄O₁₀(OH)₂Z_{1/3}·nH₂O (1)

where X = Al, Fe(III), Mn(III) or Cr(III); Y = Mg, Fe(II), Ni, Zn or Li; and Z = K, Na or Ca.

Specifically, the organically modified clay mineral can be obtained by treating, with a quaternary ammonium salt type cationic surfactant, a clay mineral which may be a natural or synthetic (in this case, an (OH) group in the formula is substituted with a fluorine) clay mineral in the montmorillonite group, such as montmorillonite, saponite or hectorite (commercial products include Veegum, Kunipia, Laponite, etc.), and sodium silicic mica or a synthetic mica known under the name of sodium or lithium taeniolite (commercial products include Dimonite, manufactured by Topy Industries, Ltd. etc.).

The quaternary ammonium salt type cationic surfactant used in this case is represented by the following general formula (2): 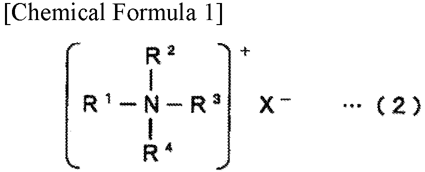 where R¹ represents a benzyl group or an alkyl group having 10 to 22 carbon atoms, R² represents an alkyl group having 10 to 22 carbon atoms or a methyl group, R³ and R⁴ represent hydroxyalkyl groups or alkyl groups having 1 to 3 carbon atoms, and X represents a halogen atom or a methylsulfate residue.

Examples of the quaternary ammonium salt type cationic surfactant include dodecyl trimethyl ammonium chloride, myristyl trimethyl ammonium chloride, cetyl trimethyl ammonium chloride, stearyl trimethyl ammonium chloride, arachyl trimethyl ammonium chloride, behenyl trimethyl ammonium chloride, myristyl dimethyl ethyl ammonium chloride, cetyl dimethyl ethyl ammonium chloride, stearyl dimethyl ethyl ammonium chloride, arachyl dimethyl ethyl ammonium chloride, behenyl dimethyl ethyl ammonium chloride, myristyl diethyl methyl ammonium chloride, cetyl diethyl methyl ammonium chloride, stearyl diethyl methyl ammonium chloride, arachyl diethyl methyl ammonium chloride, behenyl diethyl methyl ammonium chloride, benzyl dimethyl myristyl ammonium chloride, benzyl dimethyl cetyl ammonium chloride, benzyl dimethyl stearyl ammonium chloride, benzyl dimethyl behenyl ammonium chloride, benzyl methyl ethyl cetyl ammonium chloride, benzyl methyl ethyl stearyl ammonium chloride, dibehenyl dihydroxyethyl ammonium chloride, and corresponding bromides, etc., and further thereto, dipalmityl propyl ethyl ammonium methyl sulfate, etc. When carrying out the present invention, one or more of these compounds may be arbitrarily selected.

Representative examples of component (B) include dimethyl distearyl ammonium hectorite (disteardimonium hectorite), dimethyl alkyl ammonium hectorite, benzyl dimethyl stearyl ammonium hectorite, distearyl dimethyl ammonium chloride-treated aluminum-magnesium silicate, etc. Of these, dimethyl distearyl ammonium hectorite is particularly preferred. As commercial products, Bentone 27 (benzyl dimethyl stearyl ammonium chloride-treated hectorite, manufactured by Elementis Japan KK) and Bentone 38 (distearyl dimethyl ammonium chloride-treated hectorite, manufactured by Elementis Japan KK) are preferred.

The blended amount of component (B) is at least 0.2% by mass and less than 1.8% by mass, preferably 0.5% to 1.5% by mass relative to the overall amount of the cosmetic. If the blended amount of component (B) is less than 0.2%, the formation of color streaks cannot be sufficiently suppressed, and blending 1.8% by mass or more is undesirable because the viscosity becomes high and the smooth properties in use are lost.

### <(C) Oil gelling agent>

The (C) oil gelling agent (hereinafter sometimes referred to as "component (C)") can adjust the viscosity of the oil phase aside from component (B), and for example, includes dextrin fatty acid esters, hydrocarbon-styrene copolymer gelling agents, polymer compounds having urethane backbones, glyceryl fatty acid esters, amino acid gelling agents and sucrose fatty acid esters.

The dextrin fatty acid esters are esters of dextrin or reduced dextrin with a higher fatty acid, which can be used without any particular limitations as long as they are commonly used in cosmetics. A dextrin or a reduced dextrin having an average degree of polymerization of 3 to 100 is preferably used. Additionally, as the constituent fatty acids of the dextrin fatty acid esters, saturated fatty acids having 8 to 22 carbon atoms are preferably used. Specific examples include dextrin palmitate, dextrin oleate, dextrin stearate, dextrin myristate, dextrin (palmitate/2-ethylhexanoate), etc.

The hydrocarbon-styrene copolymer gelling agents refer to copolymers of at least one styrene unit with a unit selected from among butadiene, ethylene, propylene, butylene and isopropene, hydrogenated hydrocarbon copolymers, and mixtures thereof. Specific examples include mixtures of (ethylene/propylene/styrene) copolymers and (butylene/ethylene/styrene) copolymers. An example of a commercially available product is Versagel ME 2000 (manufactured by Calumet Penreco LLC).

Examples of the polymer compounds having urethane backbones include castor oil/IPDI copolymer, which is a copolymer of castor oil with isophorone diisocyanate (IPDI), etc. An example of a commercially available product is Estogel M (castor oil/IPDI copolymer (dissolved in tri(caprylic/capric)glyceride), manufactured by DKSH).

The glyceryl fatty acid esters are esterized reaction products obtained by reacting glycerin, a dibasic acid having 18 to 28 carbon atoms, and a fatty acid having 8 to 28 carbon atoms (excluding dibasic acids), which can be used without any particular limitations as long as it can be generally used in cosmetics. Specific examples include glyceryl (behenate/isostearate/eicosanedioate), glyceryl (behenate/eicosanedioate), polyglyceryl-10 (behenate/eicosanedioate), etc.

Examples of the amino acid gelling agents include N-lauroyl-L-glutamic acid dibutylamide (dibutyl lauroyl glutamide), N-2-ethylhexanoyl-L-glutamic acid dibutylamide (dibutyl ethylhexanoyl glutamide), polyamide-8, polyamide-3, etc.

As examples of the sucrose fatty acid esters, those in which the fatty acids are linear or branched, saturated or unsaturated, and have 12 to 22 carbon atoms can be favorably used. Specific examples include sucrose caprylic acid esters, sucrose capric acid esters, sucrose lauric acid esters, sucrose myristic acid esters, sucrose palmitic acid esters, sucrose stearic acid esters, sucrose oleic acid esters, sucrose erucic acid esters, etc.

As the component (C) in the present invention, one type selected from the oil gelling agents mentioned above may be used alone, or a combination of two or more types may be used.

The blended amount of component (C) is 0.05% to 5% by mass, preferably 0.1% to 3% by mass relative to the overall amount of the cosmetic. If the blended amount of component (C) is less than 0.05% by mass, there are cases in which sufficient thickening effects cannot be obtained, and if more than 5% by mass is blended, there are cases in which the viscosity becomes too high and the properties in use become poor.

Additionally, the water-in-oil emulsion cosmetic according to the present invention has a viscosity of 10,000 to 70,000 mPa·s. If the viscosity is less than 10,000 mPa s, there are cases in which the oils undergo syneresis. On the other hand, if 70,000 mPa·s is exceeded, the viscosity becomes too high, the water-in-oil emulsion cosmetic becomes heavy to spread at the time of application, and good properties in use cannot be obtained. The viscosity in the present specification is a value measured with a B-type viscometer at 30 °C.

Normally, when an ultraviolet scattering agent is further blended into a water-in-oil emulsion cosmetic containing pigments, the amount of powder in the oil phase becomes too high, an appropriate dispersion state cannot be maintained, and aggregation of the powder components tends to occur.

Since the water-in-oil emulsion cosmetic according to the present invention has excellent powder dispersibility and formulation stability due to blending the above-mentioned components (B) and (C), a (D) ultraviolet scattering agent can be further blended therein.

The (D) ultraviolet scattering agent (hereinafter sometimes referred to simply as "component (D)") blended into the cosmetic according to the present invention is not particularly limited as long as it is a powder that is used as an ultraviolet scattering agent in the cosmetics field. Specific examples include one or more types selected from among titanium oxide, zinc oxide, barium sulfate, iron oxide, talc, mica, sericite, kaolin, titanated mica, Prussian blue, chromium oxide, chromium hydroxide, silica, cerium oxide, etc. In particular, a powder having a refractive index of 1.5 or higher, for example, zinc oxide or titanium oxide, is preferably used because of the optical properties thereof. Additionally, the powder should preferably consist of fine particles having an average particle size smaller than 0.1 µm. The lower limit of the average particle size is not particularly limited, but should normally be approximately 5 nm.

Additionally, the (D) ultraviolet scattering agent may have particle surfaces that are hydrophobized. By performing a surface hydrophobization treatment, the dispersibility in oil and the water resistance are improved. Methods of surface treatment include silicone treatments with methylhydrogen polysiloxane, methyl polysiloxane, etc.; alkyl silane treatments; fluorine treatments with perfluoroalkyl phosphoric acid esters, perfluoroalcohols, etc.; amino acid treatments with N-acylglutamic acid, etc.; as well as lecithin treatments; metal soap treatments; fatty acid treatments; alkyl phosphoric acid ester treatments; etc.

The blended amount of component (D) is the amount necessary to obtain the desired ultraviolet protection effects and is not particularly limited. However, the blended amount should normally be 1% by mass or more, for example, 5% to 30% by mass, preferably 10% to 30% by mass relative to the overall amount of the cosmetic. If the blended amount is less than 5% by mass, sufficient ultraviolet protection effects become difficult to obtain, and if the blended amount is more than 30% by mass, the stability tends to become worse.

In water-in-oil emulsion cosmetics, there are cases in which a hydrophobized silica is used for the purpose of improving the texture in use of the cosmetics, such as by suppressing stickiness caused by oils. In this case, the amount of powder in the oil phase becomes too high, an appropriate dispersion state cannot be maintained, and aggregation of the powder components tends to occur.

The water-in-oil emulsion cosmetic according to the present invention has excellent powder dispersibility and formulation stability, even in a system in which a high concentration of powders is blended, due to blending the above-mentioned components (B) and (C). Thus, in the water-in-oil emulsion cosmetic according to the present invention, (E) a hydrophobized silica may be further used in order to improve the texture in use of the cosmetic.

The (E) hydrophobized silica (hereinafter sometimes referred to simply as "component (E)") that may be blended into the cosmetic according to the present invention refers to a silica (silicic anhydride) that is normally used in the cosmetics field, that has a hydrophobic particle surfaces, and that has an average particle size of 1 to 10 µm, a specific surface area of 200 to 300 m²/g, and that has an oil absorption rate of 100 to 200 ml/100 g. In particular, a porous spherical silica is preferable.

The method for hydrophobizing the particle surfaces of the (E) hydrophobized silica is not particularly limited. However, it is particularly preferable to use a silicone such as methylhydrogen polysiloxane or dimethyl polysiloxane, or a fatty acid ester such as dextrin palmitate, as the hydrophobizing agent. Such hydrophobized silicas include, for example, SA-SB-150, SA-SB-300 (Miyoshi Kasei, Inc.), etc.

Component (E) is an optional blended component in the cosmetic of the present invention and therefore does not essentially need to be blended. However, when blended, it should preferably be blended in an amount required to obtain a good texture in use of the cosmetic while also being within limits in which the blended amount does not become excessive and problems such as the feel in use becoming poor are not observed. The lower limit of the suitable blended amount of component (E) in the present invention is at least 5% by mass, preferably at least 8% by mass relative to the overall amount of the cosmetic, and the upper limit is at most 20% by mass. The range of the blended amount may be 5% to 20% by mass or 8% to 20% by mass.

Additionally, in water-in-oil emulsion cosmetics in which high concentrations of powders are blended, "powdery squeakiness" sometimes occurs. In this case, "powdery squeakiness" refers to a sense of powderiness that occurs after application, with reduced flowability in the coating film and loss of smoothness over time.

In the water-in-oil emulsion cosmetic according to the present invention, (F) a semi-solid oil may be further blended in order suppress the powdery squeakiness.

The (F) semi-solid oil (hereinafter sometimes referred to simply as "component (F)") used in the present invention refers to an oil that is normally used in the cosmetics field, that does not exhibit flowability at ambient temperature (25 °C), and that has a melting point of 30 °C to 60 °C, preferably 30 °C to 45 °C, at 1 atm.

Specific examples of the semi-solid oil include vaseline, pentaerythrityl tetra(behenate/benzoate/ethylhexanoate), dipentaerythrityl hexahydroxystearate, phytosteryl macadamiate, dimer dilinoleic acid ester, macadamia nut oil polyglyceryl-6 esters behenate, dipentaerythrityl tetra(hydroxystearate/isostearate), bis-diglyceryl polyacyladipate-2, etc. In particular, pentaerythrityl tetra(behenate/benzoate/ethylhexanoate) or dipentaerythrityl hexahydroxystearate is preferably used.

Component (F) is an optional blended component in the cosmetic of the present invention and therefore does not essentially need to be blended. However, when blended, it should preferably be blended in an amount such that the effects of the blending can be observed while also being within limits in which the blended amount does not become excessive and problems such as the feel in use becoming poor are not observed. The suitable blended amount of the component (F) in the cosmetic of the present invention is 1% to 5% by mass, preferably 1.5% to 4% by mass.

The water blended into the cosmetic according to the present invention is selected, as needed, from among ion-exchanged water, purified water, tap water, natural water, etc. The blended amount is the balance (in percentage by mass relative to the overall amount of the cosmetic) with respect to the sum of the essential components of the present invention and the other optional blended components. In general, the blended amount should preferably be approximately 5% to 40% by mass relative to the overall amount of the cosmetic.

Aside from the components described above, other optional components that are used in external preparations for the skin such as normal cosmetic products and pharmaceutical products may be appropriately blended, as needed, into the cosmetic according to the present invention within a range not compromising the objective and effects of the present invention. The other optional components include, for example, usable powders other than the aforementioned components (A), (D) and (E); oils; ultraviolet absorbing agents; non-ionic, cationic and silicone-based surfactants; lower alcohols; polyhydric alcohols; chelators; pH adjusters; anti-oxidants; fragrances; preservatives; microbicidal agents; various types of drugs, etc. However, the optional components are not limited to those indicated.

The cosmetic of the present invention can be produced in accordance with a normal method. That is, the water-in-oil emulsion cosmetic can be obtained by mixing the powder components and the oil phase components while heating as needed, thereby obtaining an oil phase component mixture in which the powder components are uniformly dispersed, adding a water phase component mixture, obtained by separately mixing and dissolving the water phase components, to the oil phase component mixture, and emulsifying the mixture with a homomixer, etc.

The cosmetic of the present invention can be prepared in a format such as a gel, a paste, a cream or a balm. In particular, the present invention is suited to forming a cream-type cosmetic filling a wide-mouthed container or a tube.

The cosmetic of the present invention may be provided as a makeup cosmetic such as a makeup base, a foundation, a concealer, a blush, an eyeshadow, a mascara, an eyeliner, an eyebrow pencil, an overcoat, a lipstick, etc.; or as a skin-care cosmetic such as a sunscreen, among which it is suited to forming a makeup cosmetic having sunscreen effects.

### EXAMPLES

Though the present invention will be explained in further detail by providing examples below, the present invention is not limited in any way by these examples. Where not specially noted, the blended amounts are indicated in percentage by mass relative to the systems in which the relevant components are blended. Before specifically explaining the respective examples, the evaluation methods that were employed will be explained.

### 1. Properties in use

Actual usage tests were performed by five expert panelists. The smoothness (spreadability) when each sample was applied to the skin was evaluated by assigning scores based on the five-level evaluation indicated below, and by calculating the average values thereof.
5: Very good
4: Good
3: Normal
2: Slightly poor
1: Poor

### (Evaluation criteria)

A: Average score of at least 4.0 points
B: Average score of at least 3.0 point and less than 4.0 points
C: Average score of at least 1.5 points and less than 3.0 points
D: Average score less than 1.5 points

### 2. Color streaks

For the cosmetic of each sample, turbine-type stirring blades were attached to a three-one motor (manufactured by Shinto Scientific Co., Ltd.) and installed so that the bottom surfaces of the stirring blades contacted the surfaces of the cosmetic of each sample, then rotated for 4 hours at 12 rpm. Thereafter, the bulk was visually observed and evaluated based on the criteria below. The fewer color streaks there are, the more the pigment is uniformly dispersed.

### (Evaluation criteria)

A: Absolutely no color streaks observed
B: Hazy color streaks within a narrow range, yet no problems in terms of outer appearance overall
C: Hazy color streaks over a wide range
D: Distinct color streaks

### 3. Formulation stability over time

The cosmetic of each example was placed at rest for four weeks at 50 °C, then visually observed for the presence or absence of syneresis (oil rising), and evaluated based on the evaluation criteria indicated below.

### (Evaluation criteria)

A: No syneresis occurred, with the surface having no roughness and being lustrous
B: No syneresis occurred, but there was some roughness on the surface
C: Small amount of transparent liquid observed on surface (slight syneresis occurred)
D: State of collected transparent liquid observed (significant syneresis occurred)

The foundation creams having the compositions listed in Table 1 below were prepared by conventional methods. The prepared samples were evaluated regarding the respective properties in accordance with the aforementioned evaluation method. The results are also shown in the table.

**[Table 1]**

| Composition | Ex 1 | Ex 2 | Ex 3 | Ex 4 | Ex 5 | Ex 6 | Com p Ex 1 | Com p Ex 2 | Com p Ex 3 | Com p Ex 4 | Ref Ex 1 | Ref Ex 2 |
|---|---|---|---|---|---|---|---|---|---|---|---|---|
| Hydrogenated didecene | 13 | 13 | 13 | 13 | 13 | 13 | 13 | 13 | 13 | 13 | 13 | 13 |
| Hydrogenated polydecene | 1 | 1 | 1 | 1 | 1 | 1 | 1 | 1 | 1 | 1 | 1 | 1 |
| Caprylyl methicone | 4 | 4 | 4 | 4 | 4 | 4 | 4 | 4 | 4 | 4 | 4 | 4 |
| Pentaerythrityl tetra(behenate/ benzoate/ethylhexa noate) | 3 | 3 | 3 | 3 | 3 | 3 | 3 | 3 | 3 | 3 | 3 | 3 |
| Ethylhexyl salicylate | 4 | 4 | 4 | 4 | 4 | 4 | 4 | 4 | 4 | 4 | 4 | 4 |
| Diisopropyl sebacate | 4 | 4 | 4 | 4 | 4 | 4 | 4 | 4 | 4 | 4 | 4 | 4 |
| Hydrogenated palm oil | 0.0 5 | 0.0 5 | 0.0 5 | 0.0 5 | 0.0 5 | 0.0 5 | 0.05 | 0.05 | 0.05 | 0.05 | 0.05 | 0.05 |
| Sorbitan sesquiisostearate | 1 | 1 | 1 | 1 | 1 | 1 | 1 | 1 | 1 | 1 | 1 | 1 |
| Isostearic acid | 1 | 1 | 1 | 1 | 1 | 1 | 1 | 1 | 1 | 1 | 1 | 1 |
| Distearyldimonium chloride | 0.1 | 0.1 | 0.1 | 0.1 | 0.1 | 0.1 | 0.1 | 0.1 | 0.1 | 0.1 | 0.1 | 0.1 |
| Palmitic acid | 0.0 5 | 0.0 5 | 0.0 5 | 0.0 5 | 0.0 5 | 0.0 5 | 0.05 | 0.05 | 0.05 | 0.05 | 0.05 | 0.05 |
| PEG-10 hydrogenated castor oil | 2.5 | 2.5 | 2.5 | 2.5 | 2.5 | 2.5 | 2 | 2 | 2.5 | 2.5 | 2.5 | 2.5 |
| Lauryl PEG-9 polydimethyl siloxy ethyl dimethicone | 3.5 | 3.5 | 3.5 | 3.5 | 3.5 | 3.5 | 3.5 | 3.5 | 3.5 | 3.5 | 3.5 | 3.5 |
| Disteardimonium hectorite | 0.9 | 1.1 | 0.8 | 0.9 | 0.9 | 0.9 | 1.8 | 2 | 1.5 | - | 1.8 | - |
| Dextrin palmitate | 1 | 1 | 1 | - | - | - | - | - | - | 3 | - | 3 |
| Mixture of (ethylene/ propylene/styrene) copolymer and (butylene/ethylene/ styrene) copolymer and hydrogenated polyisobutene (solid part 5%) | - | - | - | 2.4 (sol id part 0.1 2) | - | - | - | - | - | - | - | - |
| (Castor oil/IPDI) copolymer | - | - | - | - | 1 | - | - | - | - | - | - | - |
| Glyceryl (behenate/ eicosanedioate) | - | - | - | - | - | 0.8 | - | - | - | - | - | - |
| Hydrophobized fine-particle titanium oxide | 5 | 5 | 5 | 5 | 5 | 5 | 5 | 5 | 5 | 5 | 5 | 5 |
| Hydrophobized fine-particle zinc oxide | 10 | 10 | 10 | 10 | 10 | 10 | 10 | 10 | 10 | 10 | 10 | 10 |
| Hydrophobized silica | 10 | 10 | 10 | 10 | 10 | 10 | 10 | 10 | 10 | 10 | 10 | 10 |
| Amino acid-treated pigment-grade titanium oxide | 4 | 4 | 4 | 4 | 4 | 4 | 4 | 4 | 4 | 4 | - | - |
| Amino acid-treated yellow iron oxide | 0.9 | 0.9 | 0.9 | 0.9 | 0.9 | 0.9 | 0.9 | 0.9 | 0.9 | 0.9 | - | - |
| Amino acid-treated red iron oxide | 0.3 4 | 0.3 5 | 0.3 5 | 0.3 5 | 0.3 5 | 0.3 5 | 0.35 | 0.35 | 0.35 | 0.35 | - | - |
| Amino acid-treated black iron oxide | 0.1 8 | 0.1 8 | 0.1 8 | 0.1 8 | 0.1 8 | 0.1 8 | 0.18 | 0.18 | 0.18 | 0.18 | - | - |
| Silicone-treated pigment-grade titanium oxide | - | - | - | - | - | - | - | - | - | - | 4 | 4 |
| Silicone-treated yellow iron oxide | - | - | - | - | - | - | - | - | - | - | 0.9 | 0.9 |
| Silicone-treated red iron oxide | - | - | - | - | - | - | - | - | - | - | 0.35 | 0.35 |
| Silicone-treated black iron oxide | - | - | - | - | - | - | - | - | - | - | 0.18 | 0.18 |
| Water | 23. 78 | 23. 57 | 23. 87 | 22. 37 | 23. 77 | 23. 97 | 24.3 7 | 24.1 7 | 24.1 7 | 22.6 7 | 23.8 7 | 22.6 7 |
| Alcohol | 2 | 2 | 2 | 2 | 2 | 2 | 2 | 2 | 2 | 2 | 2 | 2 |
| Phenoxyethanol | 0.5 | 0.5 | 0.5 | 0.5 | 0.5 | 0.5 | 0.5 | 0.5 | 0.5 | 0.5 | 0.5 | 0.5 |
| EDTA-3Na | 0.2 | 0.2 | 0.2 | 0.2 | 0.2 | 0.2 | 0.2 | 0.2 | 0.2 | 0.2 | 0.2 | 0.2 |
| Glycerin | 4 | 4 | 4 | 4 | 4 | 4 | 4 | 4 | 4 | 4 | 4 | 4 |
| TOTAL | 100 | 100 | 100 | 100 | 100 | 100 | 100 | 100 | 100 | 100 | 100 | 100 |
| Viscosity (mPa·s) | 489 30 | 504 00 | 467 00 | 497 00 | 465 00 | 483 00 | 821 00 | 758 00 | 489 00 | 401 00 | 790 00 | 410 00 |
| Properties in use (smoothness) | A | B | A | A | A | A | C | C | A | B | C | A |
| Color streaks | A | A | A | A | A | A | A | A | C | C | A | A |
| Formulation stability over time | A | A | A | A | A | A | A | A | A | C | A | B |

As indicated in the table, component (C) was not blended into Comparative Examples 1 to 3, and the oil phase was thickened by only component (B). In the case in which component (C) was not blended and the blended amount of component (B) was high (the cosmetics of Comparative Examples 1 and 2), smooth properties in use were not able to be realized. In the case in which component (C) was not blended and the blended amount of component (B) was low (the cosmetic of Comparative Example 3), color streaks formed. Thus, it was not possible to realize both features.

Additionally, in the cosmetic of Comparative Example 4, in which component (B) was not blended, color streaks were formed and the formulation stability was also inferior.

Meanwhile, the cosmetics of Examples 1 to 3, containing components (B) and (C) of the present invention, and the cosmetics of Examples 4 to 6, in which component (C) has been changed to various types of oil gelling agents, all did not form color streaks, had excellent formulation stability, and were also able to realize smooth properties in use.

For reference, in Reference Example 1 and Reference Example 2 containing pigments having silicone-treated surfaces, color streaks were not formed even when component (B) and component (C) of the present invention were not blended.

From the above, it was demonstrated that component (B) and component (C) of the present invention are essential for suppressing color streaks in the case in which an amino acid-treated pigment is used.

### (Formulation Example)

A formulation example of the water-in-oil emulsion cosmetic of the present invention is indicated below. Needless to say, the present invention is not limited in any way by this formulation example and is specified by the claims. The blended amounts are all indicated as percentages by mass relative to the overall amount of the product.

**Formulation example: Foundation**

| Component name | Blended amount (% by mass) |
|---|---|
| Hydrogenated didecene | 15 |
| Pentaerythrityl tetra(behenate/benzoate/ethylhexanoate) | 3 |
| Ethylhexyl salicylate | 5 |
| Diisopropyl sebacate | 5 |
| 2-Methylphenyl 4-methoxycinnamate | 2 |
| Sorbitan sesquiisostearate | 1 |
| Isostearic acid | 1 |
| Distearyldimonium chloride | 0.1 |
| Palmitic acid | 0.05 |
| PEG-10 hydrogenated castor oil | 2.5 |
| Lauryl PEG-9 polydimethylsiloxyethyl dimethicone | 3.5 |
| Disteardimonium hectorite | 1 |
| Dextrin palmitate | 1 |
| Hydrophobized fine-particle titanium oxide | 5 |
| Hydrophobized fine-particle zinc oxide | 10 |
| Hydrophobized silica | 10 |
| Amino acid-treated pigment-grade titanium oxide | 4 |
| Amino acid-treated yellow iron oxide | 1 |
| Amino acid-treated red iron oxide | 0.3 |
| Amino acid-treated black iron oxide | 0.2 |
| Water | 22.65 |
| Alcohol | 2 |
| Phenoxyethanol | 0.5 |
| EDTA-3Na | 0.2 |
| Glycerin | 4 |
| Total | 100 |

## Claims

1. A water-in-oil emulsion cosmetic comprising:
(A) an amino acid-treated pigment;
(B) an organically modified clay mineral; and
(C) an oil gelling agent other than component (B).

2. The water-in-oil emulsion cosmetic according to claim 1, further comprising (D) an ultraviolet scattering agent.

3. The water-in-oil emulsion cosmetic according to claim 1, wherein the blended amount of component (A) is at least 4% by mass relative to the overall amount of the cosmetic.

4. The water-in-oil emulsion cosmetic according to claim 1, wherein the blended amount of component (B) is at least 0.2% by mass and less than 1.8% by mass relative to the overall amount of the cosmetic.

5. The water-in-oil emulsion cosmetic according to claim 1, further comprising (E) a hydrophobized silica.

6. The water-in-oil emulsion cosmetic according to claim 1, having a viscosity of 10,000 to 70,000 mPa·s.
